# FASCICULE DE BREVET EUROPEEN

(11) **EP 3 545 070 B1**
(45) Date de publication et mention de la délivrance du brevet: **22.07.2020**
(21) Numéro de dépôt: 17786967.4
(22) Date de dépôt: 28.09.2017
(51) Int. Cl.: C12M 1/12, C12M 1/00

(54) **PANNEAU POUR PHOTOBIOREACTEUR**
PHOTOBIOREAKTOR PANEL
PANEL FOR PHOTOBIOREACTOR

(30) Priorité: 25.11.2016 FR 1661481
(43) Date de publication de la demande: 02.10.2019
(73) Titulaire: Brochier Technologies, 69100 Villeurbanne (FR)
(72) Inventeur: BROCHIER, Cédric, 69007 Lyon (FR); PERUCHON, Laure, 69100 Villeurbanne (FR)
(74) Mandataire: Cabinet Laurent & Charras
(86) Numéro de dépôt international: PCT/FR2017/052656
(87) Numéro de publication internationale: WO 2018/096229

(56) Documents cités:
- WO-A1-2011/063214
- WO-A1-2014/148903
- WO-A1-2015/078451
- FR-A1- 2 975 709
- FR-A1- 3 029 944
- JP-A- H07 301 712
- JP-A- H08 262 232
- US-A1- 2009 003 013
- US-A1- 2011 070 632

## Description

### DOMAINE DE L'INVENTION

La présente invention a trait au domaine des dispositifs lumineux réalisés à partir de fibres optiques. L'invention trouve plus particulièrement application en tant que dispositif lumineux pour la culture de micro-organismes photosynthétiques au sein d'un photobioréacteur, notamment le dispositif lumineux étant compris dans un panneau pour photobioréacteur selon la revendication 1.

### ETAT ANTERIEUR DE LA TECHNIQUE

Dans l'industrie de la photosynthèse de micro-organismes, deux techniques sont principalement connues :
- les techniques en réacteur ouvert ; et
- les techniques en réacteur fermé.

La technique en réacteur ouvert a très vite été laissée de côté au détriment de la technique en réacteur fermé. En effet, la technique en réacteur ouvert présente de nombreux inconvénients pour pouvoir être mise en œuvre à une échelle industrielle. Un inconvénient majeur est celui de la contamination des micro-organismes photosynthétiques et ce par des sources provenant de l'environnement extérieur tel que les gaz présents dans l'air.

En contrepartie, les photobioréacteurs fermés présentent de nombreux avantages donnés à titre d'exemple et de manière non limitative :
- le maintien des micro-organismes photosynthétiques stérile ;
- la diminution des apports énergétiques ;
- l'augmentation de la productivité.

Deux catégories de réacteurs fermés ont été développées par les industriels ces dernières années, à savoir ceux qui fonctionnent à partir de la lumière naturelle provenant de l'environnement extérieur, et ceux qui fonctionnent à partir de lumière artificielle.

Une première catégorie de réacteurs fermés est munie de plaques transparentes laissant passer la lumière de manière à alimenter en énergie lumineuse le milieu de culture de microorganismes. Ce type de réacteur n'offre toutefois pas des conditions de productivité optimale à cause du faible apport lumineux au sein du milieu de culture. Pour contrecarrer ces déficiences, certains industriels ont eu l'idée de développer des dispositifs d'accumulation d'énergie lumineuse en surface des réacteurs de manière à la redistribuer au sein de l'enceinte notamment par des moyens d'acheminement tels que les fibres optiques brutes. Le document FR 2 968 094 ou WO 20147/148903 décrivent plus en détails ce principe de capture de lumière et de sa redistribution au sein du réacteur. Toutefois, l'apport en lumière semble être toujours insuffisant pour une production de masse. Par ailleurs, les conditions à l'intérieur du réacteur contribuent à la dégradation rapide du dispositif lumineux au sein du photobioréacteur.

Une deuxième catégorie de réacteurs fermés a été développée qui consiste à produire de la lumière artificielle au sein du réacteur. Pour ce faire, les panneaux au sein du réacteur sont munis de diodes électroluminescentes (LED) ou autre organes équivalents. Le document WO 2015/078451décrit un photobioréacteur pour cultiver des organismes phototrophes, comprenant un ensemble de nattes disposés à l'intérieur du récipient, lesdites nattes comprenant plusieurs premières fibres qui sont conductrices de la lumière le long de la direction longitudinale, et plusieurs secondes fibres, qui sont électriquement conductrices le long de la direction longitudinale et permettent de générer un champ électrique alternatif progressif. Les fibres sont perméables aux fluides, et dont un fluide sous la forme de la solution nutritive peut circuler lentement à travers la natte. Quand bien même le rapport surface éclairée/volume de culture, donc l'efficacité énergétique lumineuse a été augmentée significativement, ces dispositifs présentent un inconvénient important qui est celui de la dégradation des dispositifs lumineux (LED) non protégés. En effet, il a été observé que les microorganismes ont tendance à se bloquer au niveau des LED ce qui conduit à leur détérioration. Par conséquent, la culture de microorganismes photosynthétiques au sein de ces dispositifs est limitée.

Le Demandeur estime donc qu'il existe un besoin manifeste de mettre au point un dispositif de diffusion de la lumière performant pour la culture de microorganismes photosynthétique en photobioréacteurs.

Afin de remédier à ces problèmes, il propose donc un panneau pour photobioréacteur permettant de résoudre les problèmes précités.

### EXPOSE DE L'INVENTION

L'invention concerne un panneau pour photobioréacteur comprenant au moins :
- deux plaques assemblées entre elles, dont au moins une est transparente, et entre lesquelles est interposé un dispositif d'éclairage ;
- au moins deux ouvertures permettant le passage d'un fluide d'une première face vers une seconde face du panneau.

Conformément à l'invention, ce panneau se caractérise en ce que le dispositif lumineux est un textile intégrant au moins une fibre optique qui est apte à diffuser de la lumière.

Autrement dit, le Demandeur propose un panneau pour photobioréacteur qui comprend un textile lumineux muni de fibres optiques. Ces fibres optiques sont capables de diffuser la lumière et ce latéralement par rapport à leur propre longueur. En effet, lorsque la ou les fibres optiques est/sont alimentée(s) en lumière par une source lumineuse, la lumière est alors conduite le long de la fibre. Cette même lumière peut alors être diffusée partiellement ou totalement au niveau de certaines régions de la fibre optique. Les plaques de nature transparente assurent la transmission de la lumière partiellement ou totalement et ce en direction d'une région prédéterminée. En outre, les plaques permettent de protéger le dispositif lumineux de toutes contraintes extérieures (eau, micro-organismes) pouvant contribuer à sa dégradation. Egalement, les ouvertures du panneau permettent le passage d'un fluide au travers du panneau, qui permet de faire communiquer les volumes définis entre deux panneaux adjacents. Ainsi, ce panneau peut donc être mis en œuvre en tant que dispositif lumineux notamment au sein d'enceintes de culture de microorganismes photosynthétiques comme par exemple les photobioréacteurs.

En pratique, plusieurs modes de réalisation pour le panneau sont envisageables notamment en fonction :
- de l'agencement et/ou de la structure du dispositif lumineux ;
- de l'agencement des plaques de protections ;
- de l'agencement des fibres optiques au sein du tissu ;
- du mode de diffusion de la lumière ;
- de la couleur de la lumière ;
et donc plus généralement de l'application désirée.

Comme déjà expliqué, le dispositif lumineux est un textile intégrant au moins une fibre optique qui est apte à diffuser de la lumière notamment transversalement.

En pratique, ce textile peut aussi bien être réalisé sous la forme d'un tissu, d'un tricot que d'un tressé. Généralement, le textile lumineux est préférentiellement un tissu qui est composé de fils de chaine et de fils de trame agencés selon des motifs prédéterminés que l'homme du métier saura déterminer selon les applications.

Avantageusement, ce tissu est obtenu par un procédé Jacquard au cours duquel le mode de répartition des fils de chaine et/ou trame mais également celui des fibres optiques est maitrisé avec précision. Ainsi, les fibres optiques sont tissées au sein d'une âme textile de manière contigüe et repérable.

En pratique, le tissu comprend également des fils de liage permettant le maintien des fibres optiques au sein de l'âme textile tissée. Il s'agit des fils de chaine lorsque la fibre optique est insérée en trame ou des fils de trame lorsque la fibre optique est en chaîne. Toutefois, la fibre optique est préférentiellement insérée en trame et dans ce cas, les fils de liage sont des fils de chaine.

Par ailleurs, le textile présente avantageusement des fils de liage répartis sur les fibres optiques selon une armure de type satin de manière à optimiser la surface de diffusion des fibres optiques.

Le dispositif lumineux peut présenter différents agencements et ce selon les applications visées.

Selon un premier mode de réalisation préférentiel, le dispositif lumineux est un complexe de deux tissus superposés. Ces tissus peuvent être identiques ou différents selon les applications.

De manière générale, l'assemblage tissé comprend ainsi une interface de juxtaposition formée entre les surfaces des deux tissus en contact. Il présente également deux faces opposées et qui sont chacune en contact direct avec les plaques de protection respectivement une première plaque et une seconde plaque.

Selon un premier mode de réalisation, les deux tissus sont superposés l'un à l'autre et maintenus par adjonction des deux plaques de protection.

Selon un second mode de réalisation, un film adhésif thermoplastique permet d'assurer l'adhésion entre les deux tissus. Il peut être avantageusement choisi parmi le polyuréthane (PU) ou toute autre résine thermofusible à chaud dans la plage de température préconisée pour la fibre optique, soit inférieure à 80°C.

Le film adhésif présente des propriétés optiques vis-à-vis de la lumière qui sont fonction des applications visées. Selon une première variante, le film adhésif peut être transparent à la lumière. Dans ce cas, le film ne s'oppose donc pas à la lumière et la laisse passer quasiment dans son intégralité. Ainsi, la lumière diffusée par un tissu en direction du film transparent est partiellement transmise par le tissu directement en regard, pour ensuite être diffusée en direction de la zone destinée à être éclairée. Dans ces conditions, par son agencement et sa composition, le panneau réduit les pertes d'énergie lumineuse.

Selon une deuxième variante, il peut être partiellement opaque à la lumière, mais réfléchissant. Dans ce cas, le film présente préférentiellement une couleur blanche.

Avantageusement et pour plus de rigidité, les deux tissus peuvent être séparés l'un de l'autre par un élément intercalaire rigide ou semi-rigide réalisé en polymère transparent, qui est avantageusement choisi dans le groupe comprenant le polyméthacrylate de méthyle (PMMA), le polyuréthane (PU), le polycarbonate (PC), le polyvinyle de carbone (PVC), le polypropylène (PP) et l'acétate de cellulose.

En outre, l'élément intercalaire présente avantageusement une épaisseur comprise entre 100 et 500 µm, plus avantageusement entre 150 et 300 µm.

Dans ce cas, deux matériaux adhésifs sont alors préférables pour pouvoir assurer l'adhésion de chacun des tissus de chaque côté de l'élément intercalaire. En outre, le matériau adhésif et l'élément intercalaire présentent préférentiellement des propriétés adhésives compatibles de manière à assurer l'adhésion désirée.

En pratique, les fibres optiques sont avantageusement localisées au niveau des faces du tissu qui sont directement au contact des plaques de protection. En d'autres termes, les fibres optiques émergent légèrement en surface de chacune de ces faces.

Bien évidemment, l'homme du métier saura adapter l'épaisseur de chacun des tissus de manière à favoriser un mode de diffusion de la lumière plutôt qu'un autre.

Autrement dit, l'homme du métier saura choisir les paramètres appropriés pour chaque mode de diffusion de la lumière notamment en jouant sur les paramètres tels que la nature et l'épaisseur des matériaux ainsi que leurs propriétés de diffusion de la lumière, etc.

En pratique, l'épaisseur globale du panneau est avantageusement comprise entre 1 et 5 mm, plus avantageusement comprise entre 1,5 et 3 mm.

Selon un autre mode de réalisation, le dispositif lumineux consiste en un tissu monocouche avantageusement obtenu par un procédé Jacquard.

Ce tissu présente avantageusement une amure choisie parmi le satin, le sergé et le taffetas, préférentiellement une armure type satin.

Dans ce cas, les fibres optiques sont tissées au sein même de l'âme textile et par conséquent elles émergent avantageusement de part et d'autre du plan formé par l'âme textile. En outre, les fibres optiques ont tendance à émerger principalement et de manière privilégiée d'un côté du tissu plutôt que de l'autre.

En pratique, l'épaisseur de ce tissu est comprise entre 0,5 et 5 mm, avantageusement comprise entre 0.5 et 3 mm.

Comme déjà expliqué, un élément intercalaire peut également être ajouté pour renforcer la rigidité du textile intercalé entre les deux plaques de protection. Bien entendu, la plaque de protection est réalisée en un matériau présentant des propriétés optiques adéquates.

En fonction des applications, l'homme du métier saura adapter l'épaisseur du textile de manière à favoriser un mode de diffusion de la lumière plutôt qu'un autre.

Selon un troisième mode de réalisation, le dispositif lumineux est un tissu monocouche replié sur lui-même au sein duquel toutes les fibres optiques sont disposées sur les faces opposées en regard après pliage. Autrement dit, le tissu est replié sur lui-même de façon à ce que toutes les fibres, qui sont sur une seule face du tissu, se retrouvent du côté extérieur au pliage.

Comme précisé précédemment, le tissu présente avantageusement une armure choisie parmi le satin, le sergé, le taffetas, plus avantageusement le satin.

En outre, un élément intercalaire peut être inséré entre les deux portions du tissu replié de manière à renforcer la rigidité du dispositif lumineux. Comme précisé précédemment, cet élément intercalaire présente avantageusement une épaisseur comprise entre 100 et 500 µm, préférentiellement entre 150 et 300 µm. En outre, il démontre avantageusement des propriétés de transparence à la lumière, préférentiellement il transmet au minimum 80% de la lumière.

Là encore, l'homme du métier saura adapter les paramètres que sont l'épaisseur, la nature du tissu ainsi que les propriétés de diffusion lumineuse des matériaux utilisés.

Les fibres optiques sont aptes à diffuser la lumière latéralement car elles font l'objet préalablement d'un traitement de surface qui permet d'engendrer des modifications de surface.

On désigne dans la suite par « modifications de surface », toutes modifications de la géométrie et/ou des propriétés physico-chimiques de la surface des fibres optiques, obtenues par un traitement mécanique, thermique voire chimique, permettant à la lumière se propageant dans la fibre de sortir de celle-ci au niveau des modifications de surface.

Selon un mode de réalisation, le traitement de surface peut être un traitement mécanique par par abrasion. Le traitement mécanique par abrasion permet de modifier la texture de la surface des fibres optiques. Une partie de la lumière est ainsi diffusée au niveau des modifications de surface conférant aux fibres optiques des propriétés d'éclairage latéral. Ce type de traitement est avantageux puisqu'il permet de contrôler la distribution des modifications de surface le long du dispositif lumineux, en agissant sur la vitesse, le débit et la pression d'abrasion. Ainsi, il permet plus particulièrement de former un nombre de modifications de surface qui est fonction de l'intensité de la lumière au sein du dispositif et de la répartition souhaitée.

En variante, le traitement de surface peut également être réalisé au cours d'un traitement optique par rayonnement laser. Le traitement par rayonnement laser peut également permettre d'obtenir un nombre de modifications de surface fonction de l'intensité de la lumière disponible au sein du dispositif et de la longueur de fibres optiques.

Avantageusement en pratique, le nombre de modifications de surface sur la gaine des fibres optiques, par unité de longueur, est croissant sur la longueur de la fibre en se dirigeant de la première extrémité qui est en regard de la source lumineuse vers la seconde extrémité opposée. Cette distribution des modifications de surface permet de conduire un maximum de lumière tout en assurant une diffusion homogène de la lumière sur toute la longueur des fibres optiques.

Selon un mode de réalisation particulier, la fibre optique est connectée à chacune de ses extrémités à une source lumineuse. Dans ce cas, le traitement de surface peut être adapté pour :
- qu'une première source lumineuse présente à une première extrémité permette d'éclairer sur une première demi-longueur de la fibre optique ; et
- qu'une seconde source lumineuse positionnée en regard de la deuxième extrémité de la fibre optique permette d'éclairer sur la deuxième demi-longueur de la fibre optique.

Dans tous les cas, un tel traitement de surface permet d'assurer une culture des micro-organismes qui est majoritairement uniforme dans l'intégralité du volume considéré.

Plus généralement, le traitement de surface des fibres optiques peut également être adapté en fonction de la quantité de lumière diffusée dans des zones privilégiées du volume considéré. En d'autres termes, l'homme du métier saura adapter le traitement de surface des fibres optiques selon les applications.

En outre, les fibres optiques sont avantageusement réalisées en matériau polymère choisi parmi le polyméthacrylate de méthyle (PMMA) et le polycarbonate (PC).

Au sein du textile et selon les applications, les fibres optiques peuvent être regroupées ou indépendantes les unes des autres. Lorsqu'elles sont regroupées, elles peuvent l'être aussi bien en un seul et unique faisceau qu'en une pluralité de sous-faisceaux.

Les fibres optiques d'un faisceau ou de chaque sous-faisceaux peuvent être rassemblées à au moins une de leur extrémité dans une bague destinées à être associée à une source d'éclairage.

De manière générale, les fibres optiques sont alimentées par au moins une source lumineuse. La source lumineuse peut être tant naturelle (par exemple émanant du soleil) qu'artificielle selon les applications. En outre, cette source lumineuse est avantageusement externe au panneau.

Dans certains cas, la source lumineuse peut émettre une lumière blanche ou une couleur spectrale selon les applications. Dans d'autres cas, la source lumineuse peut émettre des UV, avantageusement des UV-A de longueur d'onde comprise entre 315 et 400 nm.

Il peut s'agir d'une source lumineuse unique et commune à toutes les fibres optiques. Inversement, il peut également s'agir d'une pluralité de sources lumineuses identiques ou différentes et spécifiques à chacune des fibres optiques. Dans la pratique, la ou les source(s) de lumière peuvent être choisies parmi les diodes électroluminescentes, les diodes électroluminescentes organiques (OLED) et polymériques (PLED ou P-OLED), les diodes laser ou leur combinaison.

Selon les applications, le panneau diffuse la lumière selon des directions et des sens privilégiés. Quel que soit l'agencement du dispositif lumineux comme précédemment décrit, le panneau de l'invention est apte à diffuser la lumière selon au moins un des modes suivants.

Selon un premier mode de réalisation, le panneau éclairant des deux côtés comporte deux tissus assemblés dos à dos. Chaque tissu comportant essentiellement toutes les fibres optiques sur la même face, le tissu unitaire émet la lumière d'un côté du tissu. En pratique, le tissu unitaire émet entre 70 et 90% de la lumière totale d'un côté du tissu et diffuse 10 et 30% de la lumière de l'autre côté du tissu. Le dispositif lumineux comportant deux tissus dos à dos, il diffuse la lumière des deux côtés du panneau de façon sensiblement équivalente. En pratique, le dispositif lumineux diffuse sur chacun des deux côtés entre 80% et 120% de la lumière d'un tissu unitaire, de par la lumière cumulée entre les deux tissus.

Selon une variante dans laquelle le tissu comportant une part égale de fibres optiques sur chacune de ses faces, le dispositif lumineux diffuse la lumière des deux côtés du panneau. En d'autres termes, 50% de la lumière totale est émise de chaque côté du panneau.

Selon un autre mode de réalisation, le dispositif lumineux émet de la lumière uniquement d'un côté du panneau. Dans tous les cas, une quantité très faible de lumière est diffusée de l'autre côté du panneau. Ce type de panneau est notamment intéressant pour une utilisation en tant que panneau extérieur dans un photobioréacteur. On désigne par panneau extérieur, un panneau qui contribue à la structure porteuse d'une structure telle qu'un photobioréacteur. En effet, seulement un côté du panneau est destiné à diffuser la lumière en direction du volume de culture, l'autre côté jouant le rôle de structure porteuse du photobioréacteur.

Indépendamment de la configuration du dispositif lumineux, le panneau peut être monochrome ou polychrome selon les applications. Par « panneau monochrome », on désigne un panneau qui diffuse une lumière d'une couleur unique par exemple, du blanc, du bleu ou du rouge. Réciproquement, on désigne par « panneau polychrome », un panneau qui diffuse une lumière de plusieurs couleurs différentes. Autrement dit, le panneau peut par exemple diffuser une lumière bleue et rouge simultanément. Bien entendu, la nature de la lumière émise sera fonction du choix de la source lumineuse comme déjà expliqué supra.

Selon un premier mode de réalisation, lorsque les fibres optiques sont indépendantes les unes des autres, elles peuvent soit être toutes alimentées par une source lumineuse identique, soit être chacune alimentée par une source lumineuse émettant sur des gammes de longueurs d'ondes différentes.

Selon un deuxième mode de réalisation, quand les fibres optiques sont regroupées à au moins une de leur extrémité en un ou plusieurs faisceaux qui émergent chacun dans une bague, plusieurs variantes d'éclairement sont alors possibles.

En pratique, si les fibres sont rassemblées en un seul et unique faisceau, une unique source lumineuse, de couleur quelconque, est alors nécessaire.

En revanche, lorsque les fibres optiques ont rassemblées en une pluralité de faisceaux, chaque faisceau peut être éclairé par une source lumineuse individuelle, l'ensemble de ces sources lumineuses pouvant être de couleurs identiques ou différentes selon les applications.

Pour pouvoir être mis en œuvre dans un photobioréacteur par exemple, le dispositif lumineux doit être protégé notamment de l'eau ou encore des micro-organismes. La protection peut être assurée par différents moyens décrits ci-dessous.

Dans tous les cas, le dispositif lumineux entre deux plaques de protection qui sont transparentes et surtout inertes à l'eau.

On désigne dans la suite par plaque « transparente », une plaque apte à transmettre avantageusement entre 80 et 100% de la lumière qu'elle reçoit, plus avantageusement entre 90 et 100%.

En pratique, les plaques destinées à prendre en sandwich le dispositif lumineux sont transparentes. Bien entendu et selon l'application visée, elles présentent des propriétés de transmission de la lumière pouvant être identiques ou différentes.

En outre, ces deux plaques peuvent être réalisées en verre ou en un matériau polymère plus léger que le verre. Le matériau polymère présente d'excellentes propriétés de transmission de la lumière et il est avantageusement choisi parmi le polyméthacrylate de méthyle (PMMA) et le polycarbonate (PC), préférentiellement le polyméthacrylate de méthyle (PMMA).

Par ailleurs, ces plaques sont avantageusement inertes à la matière environnante. En effet, elles permettent de protéger le dispositif lumineux, en l'espèce le textile, au cours de son utilisation. En d'autres termes, ces plaques ont pour objectif de protéger le dispositif lumineux textile contre tous les éléments présents dans le photobioréacteur.

Selon un mode de réalisation préférentiel, les plaques sont réalisées en un même matériau qui est choisi parmi les matériaux mentionnés ci-dessus. Elles transmettent préférentiellement entre 80 et 100% de la lumière incidente reçue, plus préférentiellement entre 90 et 100%, et dans l'exemple du PMMA, environ 92%.

En outre, ces plaques sont en pratique toujours solidaires l'une de l'autre et la solidarisation est assurée par collage, par vissage ou toutes autres méthodes appropriées et connues de l'homme du métier.

De manière générale, un photobioréacteur comprend une pluralité de panneaux lumineux montés en empilement au sein d'une enceinte appropriée. L'agencement des panneaux lumineux permet de définir, entre les panneaux, des zones de culture des micro-organismes.

Selon un premier mode de réalisation, une zone de culture est formée par l'espace défini entre deux panneaux lumineux successifs séparés par un cadre périphérique, par exemple du PVC, définissant un volume dans lequel s'écoule le fluide. Dans ce cas, le panneau est structurellement composé d'un dispositif lumineux pris en sandwich entre deux plaques de protection qui peuvent présenter des configurations différentes selon les applications.

La protection du dispositif lumineux peut être assurée par l'usinage d'au moins une des deux plaques de protection, de manière à former un logement apte à recevoir le dispositif lumineux. Dans une première variante, les deux plaques de protection sont usinées à l'identique pour former un logement de volume sensiblement égal à la moitié du volume nécessaire pour confiner le dispositif lumineux. Dans ce cas, l'avantage réside dans la mise en œuvre d'un procédé permettant d'obtenir des plaques identiques. Dans une autre variante, une plaque de protection usinée est placée en regard d'une plaque de protection intacte. Dans ce cas, le dispositif lumineux vient se loger dans l'emplacement formé au sein de la plaque de protection usinée. En pratique, les dimensions du dispositif lumineux sont avantageusement identiques à celles de l'emplacement.

La protection peut aussi être réalisée par l'ajout d'au moins une entretoise d'épaisseur similaire à celle du dispositif lumineux et intercalée entre les deux plaques de protection, de manière à entourer le dispositif lumineux pour isoler ce dernier du milieu extérieur au panneau.

Ces entretoises peuvent être indépendantes des plaques et maintenues en position lors de la mise en contact des deux plaques entre elles ou dépendantes des plaques par collage. Elles peuvent également être disposées soit sur une seule plaque de protection, soit sur les deux plaques de protection.

Par ailleurs et selon les applications, ces entretoises peuvent être réalisés par plusieurs segments indépendants disposés de manière jointive autour du dispositif lumineux, ou adopter la forme d'un cadre unique entourant le dispositif lumineux.

Ces entretoises peuvent comporter une rainure afin d'accueillir un joint suffisamment compressible pour assurer un contact intime et continu avec les plaques lors du serrage des plaques, dans un souci d'étanchéité. Préférentiellement, les entretoises sont réalisées en un matériau similaire à celui des plaques afin d'assurer la compatibilité entre la colle, la plaque et l'entretoise lors de l'assemblage des entretoises avec les plaques.

De manière générale, chaque entretoise ou portion d'entretoise, placée en regard de l'ouverture de l'une des plaques de protection, présente aussi une ouverture qui est identique formant ainsi une continuité permettant le passage d'un fluide d'une première face vers une seconde face du panneau.

Dans le but de fabriquer des plaques identiques, les moyens formant les entretoises sont avantageusement répartis sur les deux plaques d'un même panneau, et disposés sur les faces en regard de ces deux plaques. Chaque plaque présente préférentiellement des moyens formant les entretoises d'épaisseur identique. De la sorte, lorsque les deux plaques sont assemblées, elles sont écartées d'une distance correspondant à la somme des épaisseurs des entretoises des deux plaques. Cette distance correspond alors à l'épaisseur du dispositif lumineux.

Selon un mode de réalisation particulier, l'immobilisation du dispositif lumineux entre les deux plaques de protection peut être réalisée par combinaison de l'usinage des plaques de protection et de l'utilisation d'entretoises.

Selon un deuxième mode de réalisation, le photobioréacteur est composé de panneaux lumineux accolés les uns aux autres qui sont configurés pour délimiter un volume de passage du fluide à l'intérieur de leur volume global. Ce volume peut être défini, soit dans une des plaques de protection, soit dans une structure formant un châssis qui supporte le dispositif lumineux protégé par les plaques de protection.

Le châssis est préférentiellement transparent et présente un espace creux d'une épaisseur définissant la dimension du volume de culture. Dans ce cas, la zone de culture étant directement intégrée dans le panneau, il suffit de mettre en contact des panneaux les uns aux autres pour obtenir le photobioréacteur. Par ailleurs, on s'affranchit de la mise en place de cadre périphérique entre panneaux ce qui permet d'obtenir un empilement plus simple et plus compact.

De manière générale, le châssis présente un compartiment dans lequel vient se loger l'assemblage plaque/textile/plaque décrit précédemment. Dans la pratique, l'assemblage est disposé au sein du châssis par collage à l'aide d'une colle transparente à la lumière. Le compartiment peut être formé de différentes manières selon les applications : soit par ajout d'entretoises, soit par usinage dans l'épaisseur du châssis. Dans le cas d'entretoises rapportées, elles peuvent être réalisées, soit en un matériau identique au châssis de manière à former une structure monolithique, soit en un matériau différent conduisant à une structure hétérogène.

Par ailleurs, le passage d'un fluide d'une première face vers une seconde face du panneau peut être assuré de différentes manières notamment par ajustement de la forme du châssis.

Selon une première variante, le châssis présente deux ouvertures dans sa structure : une au niveau de sa partie supérieure et une autre au niveau de sa partie inférieure. Ces ouvertures sont avantageusement formées dans chacune des excroissances qui forment le compartiment destiné à recevoir l'assemblage. Ce panneau comprend en outre des moyens d'étanchéité notamment au niveau des interfaces entre le châssis et l'assemblage qui sont potentiellement exposées à l'eau et/ou aux algues mais également au niveau des entretoises, quand elles existent, destinées à être au contact d'un panneau adjacent.

Selon une deuxième variante, les ouvertures sont définies conjointement par les plaques de protection et les excroissances du châssis disposé en regard.

Le positionnement sensiblement central de l'assemblage par rapport aux excroissances permet de former deux ouvertures respectivement situées entre :
- l'arête supérieure de l'assemblage et la partie supérieure du châssis ;
- l'arête inférieure de l'assemblage et la partie inférieure du châssis.

Dans ce cas, les portions de l'assemblage plaque/textile/plaque, qui définissent avec le châssis les ouvertures, sont recouvertes de moyens d'étanchéité de manière à isoler le dispositif lumineux du fluide.

Le panneau peut comprendre un organe de fixation qui permet d'en assurer le maintien sur la structure porteuse du photobioréacteur. Cet organe de fixation peut être solidaire du panneau, mais également amovible. Cet organe de fixation permet avantageusement de monter le panneau sur des rails de guidage de la structure porteuse de manière à maintenir un positionnement relatif entre les panneaux au sein du photobioréacteur.

De manière générale, le dispositif lumineux n'est en pratique pas totalement confiné entre les deux plaques de protection. Ainsi, les fibres optiques et/ou les faisceaux de fibres optiques émergent au niveau des parties latérales de l'assemblage plaque/textile/plaque qui sont perpendiculaires au sens trame du tissu. Elles sont généralement dépourvues de moyens d'étanchéité puisqu'elles ne sont exposées ni à l'eau ni aux micro-organismes.

Selon un mode de réalisation particulier, le dispositif lumineux est recouvert au moins sur une face par une couche d'enduction. Cette couche d'enduction est avantageusement réalisée à partir d'un matériau polymère choisi parmi le polyuréthane, la silicone, la résine époxy, ou tous autres matériaux hydrophobes présentant des propriétés optiques adéquates.

Par ailleurs, selon l'application visée, on peut envisager un panneau dans lequel le tissu est protégé d'un côté par une plaque de protection telle que décrite ci-dessus et recouvert de l'autre côté par une couche d'enduction préférentiellement transparente et inerte vis-à-vis de l'eau.

Un deuxième aspect de l'invention concerne le procédé de fabrication du panneau décrit précédemment.

Le procédé comprend les étapes suivantes :
a) on forme un tissu, comprenant
   - des fils de chaine et de trame constituant l'âme du tissu ;
   - des fibres optiques tissées en trame et/ou en chaine au sein du tissu et
   - des fils de liage faisant partie des fils de chaine et/ou de trame, assurant le maintien des fibres optiques au sein du tissu ;
b) on effectue un traitement de surface des fibres optiques de manière à créer des modifications de surface rendant le tissu apte à émettre de la lumière ;
c) on intercale au moins un tissu préalablement formé entre au moins deux plaques de protection ;
d) on fixe les plaques de protection entre elles et on forme un panneau lumineux.

Optionnellement, le procédé peut également comprendre des étapes de regroupement en au moins un faisceau d'au moins une des extrémités flottantes des fibres optiques en vue de l'insertion au sein d'une bague. En variante, les fibres optiques peuvent être traitées avant d'être tissées.

L'étape d) de fixation peut être réalisée soit par un moyen de collage, soit par un moyen mécanique, tel que des vis par exemple. Dans ce cas, l'étanchéité du panneau est assurée par la présence de moyens de confinement décrits précédemment.

Comme déjà expliqué, le tissu lumineux peut présenter plusieurs configurations. Par conséquent, l'homme du métier saura adapter le procédé à chacune des configurations précédemment décrites.

A titre d'exemple, lorsque le dispositif lumineux est composé de deux tissus associés dos à dos, l'homme du métier saura intégrer les étapes de positionnement des tissus entre les deux plaques avec ou sans ajout d'un élément intercalaire et/ou d'un matériau adhésif tels que décrits précédemment. Il en va de même pour la configuration concernant un tissu replié sur lui-même.

Il ressort de ce qui précède que le panneau de l'invention présente de nombreux avantages et il permet notamment :
- une utilisation en photobioréacteur ;
- un éclairement homogène et satisfaisant sur l'intégralité du panneau ;
- une adaptation simple selon les applications.

### BREVE DESCRIPTION DES FIGURES

L'invention sera mieux comprise à la lecture de la description qui va suivre, donnée uniquement à titre d'exemple, et réalisée en relation avec les figures en annexe dans lesquelles :
- la figure 1 est une vue schématique éclatée d'un panneau intégrant un tissu seul selon un mode de réalisation particulier de l'invention ;
- la figure 2 est une vue schématique éclatée d'un panneau muni d'un double tissu superposés dos à dos selon un autre mode de réalisation de l'invention ;
- la figure 3 est une vue schématique éclatée d'un panneau muni d'un tissé replié sur lui-même selon la figure 1.
- la figure 4 est une vue schématique éclatée d'un panneau disposé au sein d'un châssis selon un mode de réalisation particulier de l'invention ;
- la figure 5 est un coupe schématique d'un panneau monochrome muni d'un tissu selon l'une quelconque des figure 1, 2, 3 et 4 ;
- la figure 6 est une coupe schématique d'un panneau polychrome munis d'un tissu selon l'une quelconque des figures 1, 2 ou 3 et éclairant selon un premier mode de fonctionnement ;
- la figure 7 est une coupe schématique d'un panneau polychrome munis d'un tissu selon l'une quelconque des figures 1, 2 ou 3 et éclairant selon un autre mode de fonctionnement ;
- la figure 8 est une coupe schématique d'un panneau à double connexion.

### MANIERE DE REALISER L'INVENTION

L'invention concerne donc un panneau lumineux pour photobioréacteur dont les configurations du dispositif lumineux conduisent à de nombreux mode réalisations.

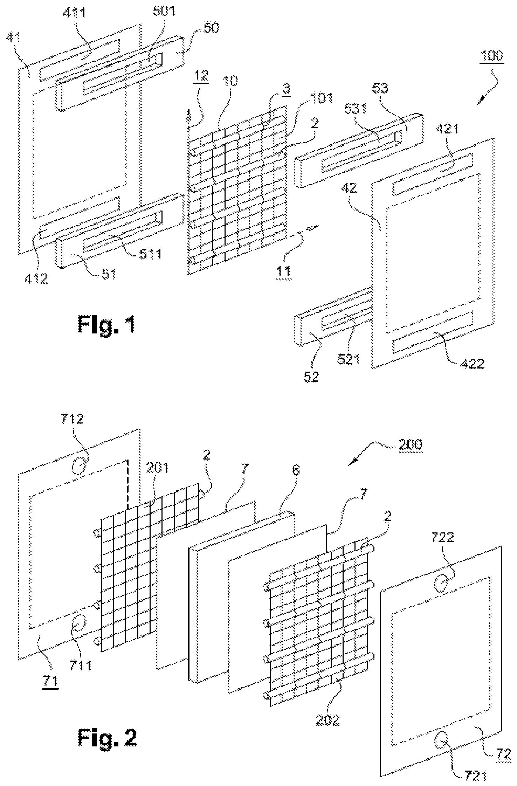

Tel que représenté sur la figure 1, le panneau **100** comprend au moins :
- deux plaques **41, 42** dont au moins une est transparente :
   - la première plaque **41** comprend deux ouvertures **411,412** et deux entretoises **50,51** présentant chacune une ouverture respectivement **501** et **511**
   - la deuxième plaque **42** comprend deux ouvertures **421,422** et également deux entretoises **52,53** présentant chacune une ouverture respectivement **521 et 531** ;
- un tissu **101** interposé entre les plaques **41,42** ; le dispositif lumineux 10 est un textile **101** intégrant au moins une fibre optique **2** apte à diffuser la lumière au travers d'au moins une plaque transparente.

Ce tissu **101** présente une amure en satin réalisée à partir de fils de chaine **12** et des fils de trame **11** en polyester Trévira® CS. Ces fils **11,12** présentent avantageusement des propriétés ignifuges. Les fils **11,12** peuvent cependant également être réalisés à partir de fils en polyamide, en fibre de verre, ou autre fibre synthétique, voire métallique. De manière générale, les fils **11,12** peuvent être réalisés à partir de fils présentant un titre compris entre 20 et 500 décitex.

Dans ce cas, les fibres optiques **2** tissées en trame sont maintenues au sein du tissu **101** par des fils de liage **3.** Les fils de liage sont positionnés sur les fibres optiques selon l'armure sélectionnée. Les fils **3** correspondent à des fils de chaine **12** qui, en plus de contribuer à la formation de l'armure, permettent le maintien de ces fibres optiques **2** au sein du tissu. Le tissu **101** présente entre 7 et 15 fibres optiques **2** par centimètre en fonction du diamètre de la fibre optique.

Le tissu **101** présente une épaisseur qui est comprise entre 0.5 et 3 mm, et qui est fonction du diamètre de la fibre optique et du mode de diffusion de la lumière.

En effet et selon les modes de réalisations, le tissu peut être apte à émettre :
- d'un seul côté du tissu **101**
- des deux côtés du tissu **101**.

L'homme du métier saura donc adapter l'épaisseur du tissu ainsi que les méthodes de tissage pour conférer à ce tissu des propriétés de diffusion de la lumière désirées selon les applications.

Un système de plaques **41,42** permet de protéger le tissu **101** lumineux. En effet, le tissu **101** est pris en sandwich entre deux plaques **41,42** chimiquement identiques réalisées préférentiellement en polyméthacrylate de méthyle (PMMA) ou en verre.

En pratique les plaques **41,42** sont destinées à être assemblées l'une à l'autre et prennent en sandwich le tissu **101** lumineux pour former le panneau **100.** La plaque **41** est munie d'une entretoise **50** montée horizontalement sur sa partie supérieure. L'orifice **501** de l'entretoise est en regard de l'ouverture **411** de la plaque **41.** La plaque **41** présente également une entretoise **51** montée horizontalement sur sa partie inférieure. Là encore, l'orifice **511** de l'entretoise **51** est positionné en regard de l'ouverture **412** de la plaque **41.**

De manière réciproque, la plaque **42** est munie d'une entretoise **52** montée horizontalement sur sa partie inférieure. L'orifice **521** de cette entretoise **52** fait face à l'ouverture **421** de la plaque **42.** La plaque **42** présente également une entretoise **53** montée horizontalement également sur sa partie inférieure. L'orifice **531** de cette entretoise **53** fait face à l'ouverture **422** de la plaque **42.**

La solidarisation des plaques **41,42** est assurée par une colle transparente compatible avec les fibres optiques. Le tissu **101** est donc partiellement confiné et bloqué entre les deux plaques **41,42** qui assurent ainsi sa protection.

Contrairement à la figure 1, le panneau **200** représenté sur la figure 2 concerne le mode de réalisation d'un dispositif lumineux comprenant deux tissus **201,202** superposés dos à dos. Dans ce cas, les tissus **201,202** sont identiques.

En outre, un élément intercalaire **6** transparent, rigide ou semi rigide est positionné à l'interface de superposition des deux tissus **201,202.** L'élément **6** est avantageusement réalisé à partir d'une feuille en polymère transparent de PMMA et confère au dispositif lumineux une rigidité renforcée.

Un matériau adhésif **7** double face réalisé en polyuréthane permet d'assurer l'adhésion des tissus **201,202** sur chacune des faces de l'élément intercalaire **6.**

Le dispositif lumineux est pris en sandwich entre une première plaque **71** présentant deux ouvertures **711,712** et une deuxième plaque **72** présentant deux ouvertures **721,722.** Lorsque les plaques **71** et **72** sont mises en contact, les ouvertures **711** et **721** et les ouvertures **712** et **722** sont disposées directement en regard en formant une continuité.

Là encore selon les modes de réalisations, le tissu peut être apte à émettre :
- d'un seul côté du panneau **200,**
- des deux côtés du panneau **200** en quantité identique.

Toutefois, le panneau **200** trouve un intérêt particulier lorsqu'il est utilisé en tant que dispositif lumineux de diffusion bilatérale de la lumière. Autrement dit, le panneau **200** peut être utilisé au sein d'un photobioréacteur pour véhiculer de la lumière simultanément sur deux sites de culture de micro-organismes localisés respectivement de chaque côté du panneau **200.**

Par ailleurs, les propriétés optiques de l'élément intercalaire **6** et du matériau adhésif **7** sont déterminantes dans le mode de diffusion du panneau final **200.**

De nouveau, l'homme du métier sera être capable de déterminer les propriétés du panneau en fonction du mode de diffusion de la lumière désiré.

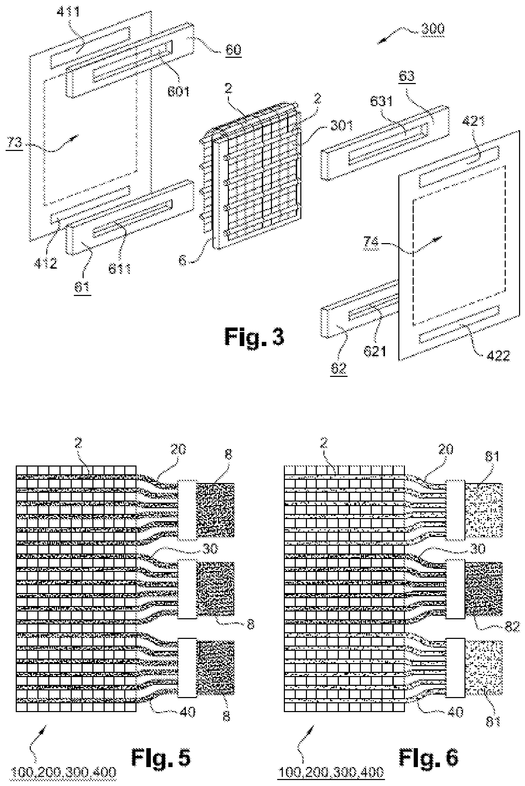

Tel que représenté sur la figure 3, le panneau **300** comprend un tissu **301** replié sur lui-même.

Comme déjà expliqué, ce tissu **301** peut intégrer au niveau de sa zone de pliure un élément intercalaire **6** tel que décrit précédemment.

Ce mode de réalisation du panneau **300** est apte à fonctionner selon le mode de diffusion bilatérale de la lumière.

En outre, les excroissances **60,61,62,63** entourent la région centrale de plus faible épaisseur, réalisée par usinage de l'épaisseur des plaques **31,32.** Elles contribuent au confinement au moins partiel du tissu **301** et permettent surtout d'assurer son isolation du milieu extérieur. Les excroissances **60** et **63** en partie haute des plaques **73** et **74** présentent chacune une ouverture **601** et **631** de plus grande largeur que les ouvertures **611** et **621** des excroissances **61** et **62** respectivement situées en partie basse des plaques **73** et **74.** Bien entendu, la configuration inverse non représentée sur les figures peut également être envisagée.

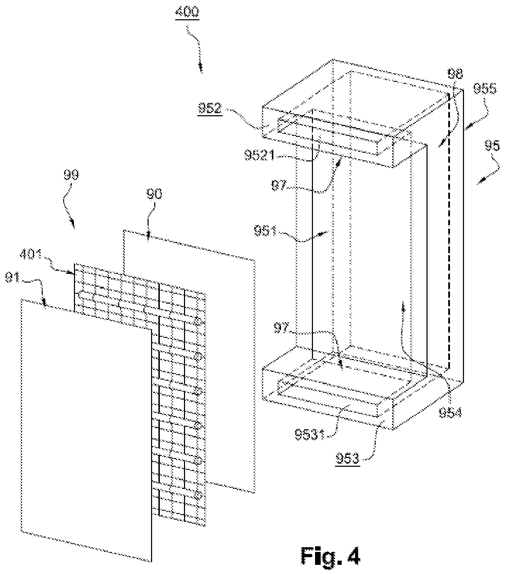

Sur la figure 4, le panneau **400** comprend un tissu unique **401** intercalé entre deux plaques **90,91** de protection en PMMA pour former un assemblage **99.** Le tissu **401** est collé à chacune des deux plaques **90,91** et ce sur tout son pourtour. Cet assemblage **99** est collé au sein d'un compartiment **951** d'un châssis **95** également réalisé en PMMA. Le collage permet d'assurer l'étanchéité au niveau des interfaces **97** entre l'assemblage **99** et le châssis **95.** Le châssis **95** présente des excroissances **952, 953** qui délimitent la hauteur du compartiment **951** destiné à recevoir l'assemblage **99.** Cette hauteur est identique à celle de l'assemblage **99.** Par ailleurs, la largeur du compartiment **951** est identique à celle de l'assemblage **99.** Ainsi, l'assemblage **99** se loge parfaitement au sein du compartiment **951** en présence de moyens appropriés pour assurer l'étanchéité. Les excroissances **952,953** étant en PMMA également ; le châssis **95** présente donc une structure monolithique. En outre, chacune des excroissances **952,953** présente une ouverture respectivement l'ouverture **9521** et l'ouverture **9531** assurant le passage d'un fluide d'une face du panneau vers l'autre face.

En outre, le châssis **95** est creux et présente une paroi **954** sur laquelle est déposé l'assemblage **99** et un volume creux **98** situé à l'arrière de la paroi **954.**

Lorsque ce panneau **400** est utilisé dans un photobioréacteur, l'eau s'écoule dans les excroissances **952,953** le long des ouvertures **9521,9531** puis poursuit son écoulement dans l'espace creux **98** du châssis **95.** Cet espace creux **98** correspond en pratique à la zone de culture des micro-organismes destinés à être cultivés.

Il a été décrit un panneau dans lequel le châssis présente une structure monolithique, toutefois l'homme du métier est capable de réaliser un châssis de structure hétérogène. Par exemple, une variante non représentée consiste en un châssis comprenant un compartiment destiné à recevoir l'assemblage et dont les excroissances sont formées par l'ajout d'entretoises réalisées à partir d'un matériau différent de celui du châssis.

Une autre variante non représentée consiste en un panneau pour lequel les ouvertures sont définies conjointement par les plaques de protection et les excroissances du châssis disposé en regard.

La figure 5 représente un panneau **100,200,300,400** selon l'invention éclairé par une source de lumière uniforme de couleur rouge.

Comme déjà expliqué, l'homme du métier saura déterminer l'agencement structurel du tissu en fonction des applications visées. En outre, l'homme du métier sera également capable de choisir la nature de la source lumineuse selon les applications.

Dans la suite, l'invention est illustrée de manière non limitative par trois variantes, différant les unes des autres par le mode de connexion aux sources lumineuses.

Tel que représenté à la figure 5, les fibres optiques **2** sont regroupées en trois faisceaux distincts **20,30,40.** Chacun de ces faisceaux **20,30,40** comprend entre 18 et 154 fibres optiques en fonction du diamètre de la fibre et de la source. Le panneau **100,200,300,400** diffuse une lumière de couleur rouge. Bien entendu, par simple changement de la nature de la source lumineuse **8,** le panneau **100,200,300,400** peut alors diffuser une lumière de couleur différente.

Tel que représenté à figure 6, les fibres optiques **2** sont toujours regroupées en trois faisceaux **20,30,40.** La différence réside dans l'alternance des couleurs diffusées par le panneau **100,200,300,400.** En effet, les faisceaux **20,40** diffusent une lumière de couleur bleue tandis que le faisceau **30** diffuse une lumière de couleur rouge. Bien entendu, l'homme du métier saura capable de jouer sur les couleurs émises par le panneau **100,200,300,400.**

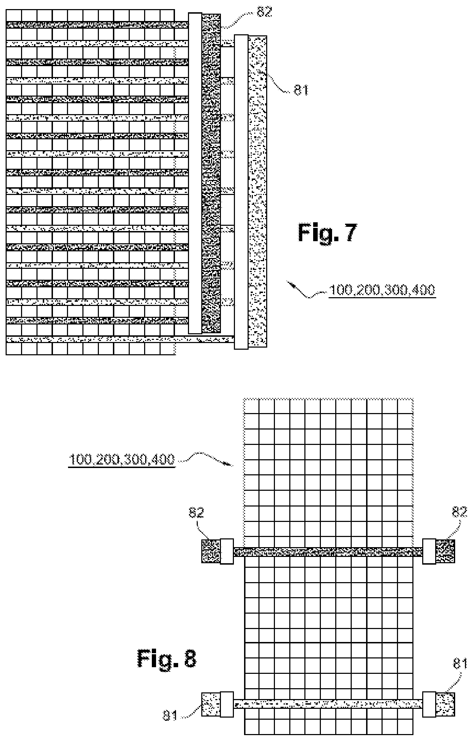

Tel que représenté à la figure 7, les fibres optiques sont connectées en deux faisceaux distincts, avantageusement en regroupant dans chaque faisceau une fibre sur deux. Les faisceaux sont alimentés respectivement par une source lumineuse **81** de couleur bleue et une source lumineuse **82** de couleur rouge. Préférentiellement, chaque faisceau regroupe entre 18 et 154 fibres optiques.

De manière générale, chaque fibre optique **2** peut être aussi bien alimentée à une ou à ces deux extrémités. Lorsque la fibre optique est éclairée à chacune de ces extrémités, 100% de la lumière émise par la source lumineuse est utilisée.

Tel que représenté sur la figure 8, le panneau **100,200,300,400** présente deux fibres optiques **2** qui sont connectés à chacune de leur extrémité respectivement à une source lumineuse **81,82.**

De manière générale, la source lumineuse **8** est avantageusement une LED qui peut émettre sur l'intégralité du spectre du visible. Préférentiellement, lorsqu'une fibre optique **2** ou un faisceau de fibres optiques **2** est alimenté par une seule source lumineuse **8,** cette source **8** présente une puissance comprise entre 3 et 10W, avantageusement voisine de 10W. Inversement, lorsqu'une fibre optique **2** ou un faisceau de fibres optiques **2** est éclairé à chacune de ces extrémités par une source lumineuse **8,** dans ce cas la source **8** présente une puissance comprise entre 1 et 3W.

Généralement, la distance qui sépare les différentes sources lumineuses **8** qui alimentent les fibres optiques **2** ou les faisceaux de fibres optiques **2** est comprise entre 1 et 10 cm, la distance étant déterminée en fonction du diamètre de la fibre et de la source.

## Revendications

1. Panneau (100,200,300,400) pour photobioréacteur comprenant au moins :
- deux plaques (41, 42,71, 72, 73, 74, 90, 91) assemblées entre elles, dont au moins une est transparente, et entre lesquelles est interposé un dispositif d'éclairage (10) ;
- au moins deux ouvertures (411, 412, 421, 422, 711, 712, 721, 722, 9521, 9531) permettant le passage d'un fluide d'une première face vers une seconde face du panneau (100,200,300,400) et
***caractérisé* en ce que** ledit dispositif lumineux est un textile (101,201,202,301,401) intégrant au moins une fibre optique (2) apte à diffuser la lumière au travers de ladite au moins une plaque transparente.

2. Panneau selon la revendication 1, ***caractérisé* en ce que** ledit textile est un tissu (101) monocouche.

3. Panneau selon la revendication 1 ou 2, ***caractérisé* en ce que** ledit textile est un tissu monocouche (301) replié sur lui-même au sein duquel toutes les fibres optiques (2) sont disposées sur les faces opposées en regard après pliage.

4. Panneau selon la revendication 1, ***caractérisé* en ce que** ledit textile est un complexe de deux tissus (201,202) superposés qui sont identiques.

5. Panneau selon la revendication 4, ***caractérisé* en ce que** lesdits deux tissus sont séparés l'un de l'autre par un élément intercalaire (6) rigide réalisé en polymère transparent, qui est avantageusement choisi dans le groupe comprenant le polyméthacrylate de méthyle (PMMA), le polyuréthane (PU), le polycarbonate (PC), le polyvinyle de carbone (PVC), le polypropylène (PP) et l'acétate de cellulose.

6. Panneau selon l'une des revendications 1 à 5, ***caractérisé* en ce que** ledit textile (101,201,202,301,401) diffuse de la lumière de chaque côté dudit panneau.

7. Panneau selon la revendication 6, ***caractérisé* en ce que** ledit textile (101,201,202,301,401) diffuse entre 70 et 90% de la lumière totale d'un côté dudit panneau, et entre 10 et 30% de l'autre côté dudit panneau.

8. Panneau selon la revendication 7, ***caractérisé* en ce que** ledit textile (101,201,202,301,401) diffuse 50% de la lumière totale de chaque côté dudit panneau.

9. Panneau selon l'une des revendications 1 à 8, ***caractérisé* en ce que** lesdites fibres optiques (2) sont regroupées à une de leur extrémité en au moins un faisceau de fibres optiques (2) qui émerge dans une bague, ledit faisceau étant éclairé par au moins une source lumineuse (8).

10. Panneau selon la revendication 1 à 9, ***caractérisé* en ce que** lesdites plaques sont réalisées en un matériau choisi parmi le polyméthacrylate de méthyle, le polycarbonate, le verre, avantageusement le polyméthacrylate de méthyle.

11. Panneau selon l'une des revendications 1 à 10, **caractérisé en ce que** les plaques sont identiques et présentent chacune deux ouvertures.

12. Panneau selon l'une des revendications 1 à 10, ***caractérisé* en ce qu'**il comprend un châssis (95) sur lequel sont solidarisées les deux plaques entre lesquelles est interposé le dispositif lumineux.

13. Panneau selon la revendication 12, ***caractérisé* en ce que** ledit châssis présente deux ouvertures (9521, 9531).

14. Panneau selon la revendication 12, ***caractérisé* en ce que** deux ouvertures sont délimitées entre respectivement :
- l'arête supérieure des plaques et la partie supérieure du châssis ;
- l'arête inférieure des plaques et la partie inférieure du châssis.

## Patentansprüche

1. Panel (100, 200, 300, 400) für einen Fotobioreaktor, umfassend mindestens:
- zwei zusammengebaute Platten (41, 42, 71, 72, 73, 74, 90, 91), von denen mindestens eine transparent ist und zwischen die eine Beleuchtungsvorrichtung (10) eingesetzt ist;
- mindestens zwei Öffnungen (411, 412, 421, 422, 711, 712, 721, 722, 9521, 9531), die es ermöglichen, dass ein Medium von einer ersten Seite zu einer zweiten Seite des Panels (100, 200, 300, 400) fließt ***und dadurch gekennzeichnet*, dass** es sich bei der Beleuchtungsvorrichtung um einen Stoff (101, 201, 202, 301, 401)) mit mindestens einer optischen Faser (2) handelt, die das Licht durch die mindestens eine transparente Platte streuen kann.

2. Panel nach Anspruch 1, ***dadurch gekennzeichnet, dass*** es sich bei diesem Stoff um ein einlagiges Gewebe (101) handelt.

3. Panel nach Anspruch 1 oder 2, ***dadurch gekennzeichnet, dass*** es sich bei dem genannten Stoff um ein einlagiges Gewebe (301) handelt, das auf sich zurückgefaltet ist und in dessen Inneren optische Fasern (2) auf den entgegengesetzten Seiten, die nach dem Falten einander gegenüber liegen, angeordnet sind.

4. Panel nach Anspruch 1, ***dadurch gekennzeichnet, dass*** es sich bei diesem Stoff um einen Komplex aus zwei übereinandergelegten Geweben (201, 202) handelt, die identisch sind.

5. Panel nach Anspruch 4, ***dadurch gekennzeichnet, dass*** die beiden Gewebe voneinander durch ein starres Zwischenelement (6) getrennt sind, das aus einem transparent Polymer besteht, das vorteilhafterweise ausgewählt wird aus der Gruppe mit Polymethylmethacrylat (PMMA), Polyurethan (PU), Polycarbonat (PC), Polyvinylchlorid (PVC), Polypropylen (PP) und Zelluloseacetat.

6. Panel nach einem der Ansprüche 1 bis 5, ***dadurch gekennzeichnet, dass*** dieser Stoff (101, 201, 202, 301, 401) das Licht beiderseits dieses Panels verteilt.

7. Panel nach Anspruch 6, ***dadurch gekennzeichnet, dass*** dieser Stoff (101, 201, 202, 301, 401) zwischen 70 und 90% des gesamten Lichts auf einer Seite dieses Panels verteilt und zwischen 10 und 30% auf der anderen Seite dieses Panels.

8. Panel nach Anspruch 7, ***dadurch gekennzeichnet, dass*** dieser Stoff (101, 201, 202, 301, 401) 50% des gesamten Lichts auf jeder Seite dieses Panels verteilt.

9. Panel nach Anspruch nach einem der Ansprüche 1 bis 8, ***dadurch gekennzeichnet, dass*** diese optischen Fasern (2) an einem ihrer Enden in mindestens einem Bündel optischer Fasern (2) zusammengefasst sind, die aus einem Ring ragen und dieses Bündel von mindestens einer Lichtquelle beleuchtet wird.

10. Panel nach Anspruch 1 bis 9, ***dadurch gekennzeichnet, dass*** diese Platten aus einem Material ausgeführt sind, bei dem es sich um Polymethylmethacrylat, Polycarbonat oder Glas handeln kann, vorzugsweise Polymethylmethacrylat.

11. Panel nach einem der Ansprüche 1 bis 10, **dadurch gekennzeichnet, dass** die Platten identisch sind und jeweils zwei Öffnungen aufweisen.

12. Panel nach einem der Ansprüche 1 bis 10, ***dadurch gekennzeichnet, dass*** es einen Rahmen (95) enthält, auf dem die beiden Platten befestigt sind, zwischen denen die Beleuchtungsvorrichtung angeordnet ist.

13. Panel nach Anspruch 12, ***dadurch gekennzeichnet, dass*** dieser Rahmen zwei Öffnungen (9521, 9531) aufweist.

14. Panel nach Anspruch 12, ***dadurch gekennzeichnet, dass*** die beiden Öffnungen begrenzt sind zwischen jeweils:
- der oberen Kante der Platten und dem oberen Teil des Rahmens;
- der unteren Kante der Platten und dem unteren Teil des Rahmens.

## Claims

1. A panel (100, 200, 300, 400) for photobioreactor comprising at least:
- two plates (41, 42, 71, 72, 72, 74, 90, 91) assembled to each other, at least one of which is transparent, and between which a lighting device (10) is inserted;
- at least two openings (411, 412, 421, 422, 711, 712, 721, 722, 9521, 9531) allowing the passage of a fluid from a first surface towards a second surface of the panel (100, 200, 300, 400); and
**characterized in that** the lighting device is a textile (101, 201, 202, 301, 401) incorporating at least one optical fiber (2) able to diffuse light through at least one transparent plate.

2. The panel according to claim 1, **characterized in that** said textile is a single-layer fabric (101).

3. The panel according to claim 1 or 2, **characterized in that** the textile is a single-layer fabric (301) folded on itself within which all the optical fibers (2) are arranged on the opposite facing surfaces after folding.

4. The panel according to claim 1, **characterized in that** said textile is a complex of two identical superimposed fabrics (201, 202).

5. The panel according to claim 4, **characterized in that** said two fabrics are separated from each other by a stiff spacing element (6) made of transparent polymer, which is advantageously chosen from the group comprising polymethylmethacrylate (PMMA), polyurethane (PU), polycarbonate (PC), polyvinyl carbonate (PVC), polypropylene (PP) and cellulose acetate.

6. The panel according to one of claims 1 to 5, **characterized in that** said textile (101, 201, 202, 301, 401) diffuses light from each side of said panel.

7. The panel according to claim 6, **characterized in that** said textile (101, 201, 202, 301, 401) diffuses between 70 and 90% of the total light from one side of said panel, and between 10 and 30% from the other side of said panel.

8. The panel according to claim 7, **characterized in that** said textile (101, 201, 202, 301, 401) diffuses 50% of the total light from each side of said panel.

9. The panel according to one of claims 1 to 8, **characterized in that** said optical fibers (2) are grouped at one end thereof into at least one bundle of optical fibers (2) which emerge in one ring, where said bundle is lit by at least one light source (8).

10. The panel according to one of claims 1 to 9, **characterized in that** said plates are made of a material chosen among polymethylmethacrylate, polycarbonate, and glass, advantageously polymethylmethacrylate.

11. The panel according to one of claims 1 to 10, **characterized in that** the plates are identical and have two openings each.

12. The panel according to one of claims 1 to 10, **characterized in that** it comprises a chassis (95) on which the two plates are secured and between which the lighting device is inserted.

13. The panel according to claim 12, **characterized in that** said chassis has two openings (9521, 9531).

14. The panel according to claim 12, **characterized in that** two openings are defined between respectively:
- the upper edge of the plates and the upper part of the chassis;
- the lower edge of the plates and the lower part of the chassis.
